(19) Europäisches Patentamt
European Patent Office
Office européen des brevets

(11) **EP 1 687 788 B1**

(12) **EUROPÄISCHE PATENTSCHRIFT**

(45) Veröffentlichungstag und Bekanntmachung des Hinweises auf die Patenterteilung:
**05.03.2008 Patentblatt 2008/10**

(21) Anmeldenummer: **04797874.7**

(22) Anmeldetag: **13.11.2004**

(51) Int Cl.:
**G08B 21/06** (2006.01)

(86) Internationale Anmeldenummer:
**PCT/EP2004/012888**

(87) Internationale Veröffentlichungsnummer:
**WO 2005/059857 (30.06.2005 Gazette 2005/26)**

(54) **VERFAHREN UND COMPUTERPROGRAMM ZUM ERKENNEN VON UNAUFMERKSAMKEITEN DES FAHRERS EINES FAHRZEUGS**

METHOD AND COMPUTER PROGRAMME FOR RECOGNITION OF INATTENTIVENESS IN THE DRIVER OF A VEHICLE

PROCEDE ET PROGRAMME INFORMATIQUE PERMETTANT DE DETECTER LES INATTENTIONS D'UN CONDUCTEUR DE VEHICULE

(84) Benannte Vertragsstaaten:
**DE ES FR GB IT SE**

(30) Priorität: **26.11.2003 DE 10355221**

(43) Veröffentlichungstag der Anmeldung:
**09.08.2006 Patentblatt 2006/32**

(73) Patentinhaber: **Daimler AG
70327 Stuttgart (DE)**

(72) Erfinder:
• **HENTSCHEL, Elisabeth
71106 Magstadt (DE)**
• **GALLEY, Lars, Arnim
10997 Berlin (DE)**
• **KUHN, Klaus-Peter
73655 Plüderhausen (DE)**
• **NEUMERKEL, Dietmar
13509 Berlin (DE)**

(56) Entgegenhaltungen:
**EP-A- 0 147 539          JP-A- 7 093 678
US-A- 5 745 031**

EP 1 687 788 B1

**Beschreibung**

Die Erfindung betrifft ein Verfahren und ein Computerprogramm zum Erkennen, wann der Fahrer eines Fahrzeugs, insbesondere eines Kraftfahrzeugs Kfz, während des Betriebs des Fahrzeugs unaufmerksam wird. Darüber hinaus betrifft die Erfindung einen Datenträger mit einem derartigen Computerprogramm.

Aus dem Stand der Technik sind verschiedene Vorschläge bekannt, eine Unaufmerksamkeit eines Kraftfahrzeugführers, insbesondere Einschlaftendenzen des Kraftfahrzeugführers zu erfassen.

Ein entsprechender Vorschlag ist zum Beispiel in der deutschen Offenlegungsschrift DE 198 18 239 A1 offenbart. Die dort offenbarte Vorrichtung zur Einschlafwarnung eines Kraftfahrzeugführers umfasst zum einen eine Fahrzeugumgebungserkennungs-Einrichtung zum Erfassen eines Ist-Fahrstils des Kraftfahrzeugführers. Darüber hinaus umfasst die dort beschriebene Vorrichtung eine Einrichtung zur Erfassung eines Referenzfahrstils, wobei insbesondere detektiert wird, wie viel laterale Eigenbewegungen der Kraftfahrzeugführer gewöhnlich in seinem Fahrstil hat. Schließlich umfasst die offenbarte Vorrichtung eine Vergleichslogik zum Vergleichen des Referenzfahrstils mit dem aktuellen Ist-Fahrstil, um je nach Maßgabe durch das Ergebnis dieses Vergleichs eine Warnung an den Kraftfahrzeugführer auszugeben.

Weiterhin ist aus der US-Patentschrift US 6,061,610 ein Verfahren und eine Vorrichtung zum Bestimmen der Belastung des Fahrers eines Kraftfahrzeugs bekannt. Dieses Verfahren sieht vor, dass der Lenkradwinkel des Kraftfahrzeugs zunächst erfasst wird, um daraus Vorhersagefehler für den Lenkradwinkel bei Steuerung des Kraftfahrzeugs durch den Fahrer zu generieren. Es wird dann eine Verteilung dieser Vorhersagefehler berechnet, um diese Verteilung mit einer anderen Verteilung von Vorhersagefehlern des Lenkradwinkels zu vergleichen, welche nicht das reale Lenkverhalten des Fahrers, sondern ein vorgegebenes Lenkverhalten eines stressfreien beziehungsweise entspannten Fahrers repräsentiert. Das Ergebnis dieses Vergleiches repräsentiert dann die aktuelle Belastung des Fahrers beim Fahren des Kraftfahrzeugs.

Schließlich ist aus der deutschen Offenlegungsschrift DE 25 46 345 ein Fahrerwarngerät zur Warnung von Kraftfahrzeugfahrern, bevor sie einschlafen bekannt. Das Gerät erfasst die Lenkbewegung des Fahrers des Kraftfahrzeugs, wobei vorausgesetzt wird, dass bei wachem Fahrer das Lenkrad auch bei einer Geradeausfahrt nicht gänzlich ruhig gehalten wird, sondern dass auch dann ständig wenn auch noch so kleine Lenkbewegungen stattfinden. Wenn das Fahrerwarngerät ein Ausbleiben dieser Lenkbewegungen auch über ein einstellbares Zeitintervall hinaus feststellt, so schließt das Fahrerwarngerät daraus, dass der Fahrer eingeschlafen ist oder zumindest droht einzuschlafen und es warnt dann den Fahrer durch Ausgabe eines Signals.

Dem in der zuletzt genannten Offenlegungsschrift DE 25 46 345 offenbarten Fahrerwarngerät haftet der Nachteil an, dass die Erkennung, wann der Fahrer eines Fahrzeugs unaufmerksam wird, alleine aufgrund der Detektion einer Lenkruhephase erfolgt und deshalb nur recht vage und unzuverlässig möglich ist.

In der EP 0147 539 A2 und in der JP 07 093678 A sind ein Verfahren mit den Merkmalen des Oberbegriffs beschrieben. Dieses Verfahren ist gekennzeichnet durch folgende Schritte: Erkennen einer sich an die Lenkruhephase anschließenden Lenkaktion und Ermitteln der Größe der Ausprägung dieser Lenkaktion durch Auswerten der zeitlichen Änderung des Lenkradwinkels und Ermitteln eines Maßes für die Schwere der Unaufmerksamkeit des Fahrers beim Lenken des Fahrzeugs durch Bewerten des Ergebnisses einer Verknüpfung der Ausprägung der Lenkruhephase und der Ausprägung der Lenkaktion.

Vorteilhafterweise unterscheidet dieses Verfahren - anders als der zuvor angeführte Stand der Technik - beim Erkennen einer Unaufmerksamkeit des Fahrers zwischen einer Lenkruhephase und einer sich bei Vorliegen eines Zustandes der Unaufmerksamkeit typischerweise daran anschließenden mehr oder weniger hektischen Lenkaktion. Ein Zustand der Unaufmerksamkeit wird also überhaupt nur dann angenommen, wenn sowohl die Lenkruhephase wie auch die anschließende Lenkaktion in Kombination miteinander erkannt werden; im Umkehrschluss bedeutet dies, dass das alleinige Erkennen einer Lenkruhephase oder einer Lenkaktion nicht ausreichend ist, um auf eine Unaufmerksamkeit des Fahrers zu schließen. Für die Ermittlung des Maßes der Schwere der Unaufmerksamkeit werden die festgestellten Ausprägungen von sowohl der Ruhephase wie auch der Lenkaktion miteinander verknüpft und das Ergebnis dann bewertet.

Ausgehend von diesem Stand der Technik ist es deshalb die Aufgabe der Erfindung, ein Verfahren und ein entsprechendes Computerprogramm zum Erkennen, wann der Fahrer eines Fahrzeugs unaufmerksam wird, sowie einen Datenträger mit diesem Computerprogramm bereitzustellen, welche ein zuverlässigeres Erkennen einer eventuellen Unaufmerksamkeit des Fahrers ermöglichen.

Diese Aufgabe wird durch das in Patentanspruch 1 beanspruchte Verfahren gelöst.

In modernen Fahrzeugen, insbesondere Kraftfahrzeugen, ist ein Sensor zur Erfassung des Lenkradwinkels x normalerweise ohnehin vorhanden. Für die Realisierung des beschriebenen Verfahrens bedarf es deshalb vorteilhafterweise grundsätzlich keines zusätzlichen Sensors.

In der Beschreibung werden zwei verschiedene Ausführungsbeispiele zur Realisierung der Erfindung aufgezeigt. Das erste Ausführungsbeispiel wird durch die Patentansprüche 4 - 7 und das zweite Ausführungsbeispiel durch die Patentansprüche 8 - 13 repräsentiert. Bei dem zweiten Ausführungsbeispiel ist grundsätzlich eine eigene Art der Ermittlung der Ausprägung der Lenkruhephase vorgesehen; allerdings kann diese Ermittlung auch gemäß dem ersten Ausführungsbeispiel erfolgen.

Weitere Ausgestaltungen des Verfahrens, gelten sowohl für das erste wie auch für das zweite Ausführungsbeispiel gleichermaßen.

Vorteile des ersten Ausführungsbeispiels

Die Berechnung der Ausprägung der Lenkaktion alleine durch Bildung einer Lenkradwinkelvarianz würde lediglich das Lenkverhalten des Fahrers zu einem Zeitpunkt repräsentieren. Gerade für eine zuverlässige Aussage über das Vorhandensein von Unaufmerksamkeiten ist es jedoch wichtig, auch Änderungen des Lenkverhaltens über der Zeit zu berücksichtigen. Diesem Aspekt wird erfindungsgemäß dadurch Rechnung getragen, dass in die Bildung des Varianz-Verhältnisses zwei Lenkradwinkelvarianzen einfließen, welche jeweils die Lenkaktion des Fahrers zu unterschiedlichen Zeitpunkten repräsentieren, wobei die beiden Zeitpunkte um ein Zeitintervall $\Delta t$ zueinander versetzt liegen.

Vorteilhafterweise kann das erfindungsgemäß berechnete Varianz-Verhältnis bereits als Maß für die Schwere der Unaufmerksamkeit des Fahrers beim Lenken des Fahrzeugs zum Zeitpunkt $t_1$ interpretiert werden; eine Unaufmerksamkeit des Fahrers ist insbesondere dann gegeben, wenn dieses Varianz-Verhältnis einen Wert größer 1 hat.

Vorteile des zweiten Ausführungsbeispiels

Im Unterschied zu dem ersten Ausführungsbeispiel werden bei dem zweiten Ausführungsbeispiel wesentlich weniger Parameter zur Beurteilung der Unaufmerksamkeit des Fahrers herangezogen. Deshalb ist es weniger speicherintensiv. Außerdem ist es aufgrund der Verwendung wesentlich einfacherer Algorithmen leichter beherrschbar und in Echtzeit implementierbar. Insgesamt ist es deshalb für den praktischen Einsatz im Fahrzeug gut geeignet.

So ist es von Vorteil, dass eine Beurteilung der Ausprägung der Lenkruhephase, das heißt ihrer Zeitdauer vorzugsweise lediglich durch Auswerten des Lenkradwinkels und die Ausprägung der Lenkaktion vorzugsweise lediglich durch Erfassen des maximal auftretenden Gradienten des Lenkradwinkels ermittelt wird. Eine Berechnung beziehungsweise Auswertung auf Basis der Varianzfunktion ist deshalb entbehrlich.

Die Verknüpfung der Ausprägung der Lenkruhephase mit der Ausprägung der Lenkaktion zum Ermitteln eines Maßes für die Schwere der Unaufmerksamkeit des Fahrers erfolgt bei dem zweiten Ausführungsbeispiel durch einen mehrdimensionalen Operator. Um unnötigen Rechenaufwand zu sparen, wird diese Verknüpfung jedoch vorzugsweise nur dann durchgeführt, wenn sowohl die Lenkruhephase wie auch die erwartete anschließende Lenkaktion jeweils mit einer vorbestimmten Mindestausprägung vorkommen. Sollte die Lenkruhephase oder die Lenkaktion nicht ausreichend stark ausgeprägt sein, so wird erfindungsgemäß angenommen, dass sich der Fahrer nicht in einem Zustand der Unaufmerksamkeit befindet.

Vorteile der Ausgestaltungen des erfindungsgemäßen Verfahrens, die beiden Ausführungsbeispielen gemeinsam sein können.

Vorteilhafterweise lassen sich das Ergebnis der Verknüpfung aus dem ersten oder zweiten Ausführungsbeispiel, das heißt das Varianz-Verhältnis oder das Ergebnis der Operator-Verknüpfung mit Hilfe der Sigmoid-Funktion auf einen Wahrscheinlichkeitswert abbilden. Auf diese Weise ist es möglich, eine Wahrscheinlichkeit zwischen 0 und 100 % dafür anzugeben, dass der Fahrer zum Zeitpunkt $t_1$ beim Lenken des Fahrzeugs unaufmerksam war.

In einer weiteren vorteilhaften Ausgestaltung ermöglicht das beanspruchte Verfahren auf Basis des zuvor ermittelten Wahrscheinlichkeitswertes eine Aussage darüber, mit welcher Wahrscheinlichkeit das Verhalten des Fahrers einer bestimmten, von einer Vielzahl vorgegebener und geeignet ausgewählter Mündigkeitsstufen zuzuordnen ist. Eine derartige Zuordnung erfolgt erfindungsgemäß immer unter Berücksichtigung des aktuell erfassten Lenkradwinkels.

Vorteilhafterweise kann diese Zuordnung zu den vorgegebenen Müdigkeitsstufen dadurch präzisiert werden, dass neben dem Lenkradwinkel als erstem Indikator auch weitere beobachtbare Indikatoren für die Unaufmerksamkeit des Fahrers, wie zum Beispiel dessen Lidschlussverhalten oder dessen Reaktionszeit berücksichtigt werden.

Es ist weiterhin von Vorteil, dass die Einschätzung über die Müdigkeit des Fahrers außerdem dadurch präzisiert werden kann, dass für diese Einschätzung nicht lediglich die aktuell erfassten Werte von insbesondere den genannten Indikatoren, sondern auch die in der jüngeren Vergangenheit vorgenommenen Müdigkeitseinstufungen berücksichtigt werden. Anders ausgedrückt ermöglicht diese Vorgehensweise eine Plausibilitätsprüfung der neuen Einschätzung unter Berücksichtigung der Tatsache, dass die Müdigkeit des Fahrers kein Phänomen ist, welches plötzlich auftritt und wieder verschwindet, sondern welches sich vielmehr nur stetig im Zeitablauf verändert.

Vorteilhafterweise ermöglicht das beanspruchte Verfahren nicht nur, wie bisher beschrieben, einen Rückschluss auf die Müdigkeit des Fahrers als Ursache für die detektierte Unaufmerksamkeit. Vielmehr ermöglicht es auch einen Rückschluss auf andere Ursachen für die detektierte Unaufmerksamkeit, welche zum Beispiel in einem geführten Gespräch mit einem Beifahrer oder der Bedienung einer Vorrichtung, zum Beispiel des Radios oder des Handschuhfachs im Fahrzeug, liegen können.

Um die Zuverlässigkeit einer Aussage über die Aufmerksamkeit beziehungsweise Unaufmerksamkeit des Fahrers des Fahrzeugs zu erhöhen, ist es empfehlenswert, nicht lediglich ein Ergebnis der Verknüpfung nach dem ersten oder zweiten Ausführungsbeispiel auszuwerten, sondern stattdessen diese Aussage auf eine Vielzahl derartiger Verknüpfungsergebnisse zu stützen. Dabei kann diese Mehrzahl sowohl lediglich aus Ergebnissen des ersten oder des zweiten Ausführungsbeispiels, jedoch auch aus einer Mischung von Ergebnissen des ersten und des zweiten Ausführungsbeispiels bestehen. Konkret kann eine Aussage über die Unaufmerksamkeit des Fahrers dadurch zuverlässiger getroffen werden, dass jedes aus einer Verknüpfung resultierende Ergebnis mit einem zugeordneten Gewichtungsfaktor gewichtet wird, um dann aus der vorliegenden Mehrzahl von gewichteten Verknüpfungsergebnissen durch mathematische Mittelwertbildung letzten Endes ein gemitteltes Verknüpfungsergebnis zu gewinnen. Dieses gemittelte Verknüpfungsergebnis repräsentiert dann im Vergleich zu einem nicht gemittelten Verknüpfungsergebnis ein zuverlässigeres Maß für die Schwere der Unaufmerksamkeit des Fahrers beim Lenken des Fahrzeugs zu einem bestimmten Zeitpunkt.

Schließlich ist es von Vorteil, wenn insbesondere der Fahrer des Fahrzeugs über die erkannte Unaufmerksamkeit in Form eines optischen oder akustischen Warnhinweises informiert wird.

Weitere vorteilhafte Ausgestaltungen des Verfahrens sind Gegenstand der abhängigen Ansprüche.

Die oben genannte Aufgabe der Erfindung wird weiterhin durch ein Computerprogramm zur Durchführung des beschriebenen Verfahrens, durch einen Datenträger mit dem Computerprogramm sowie durch ein Steuergerät zum Durchführen des beschriebenen Verfahrens gelöst. Die Vorteile dieser Lösungen entsprechen den oben mit Bezug auf das beschriebene Verfahren genannten Vorteilen.

Vorteilhafterweise braucht dieses Computerprogramm zumindest für einzelne Fahrzeugtypen nur einmal programmiert zu werden und kann dann in allen Fahrzeugen einer entsprechenden Serie implementiert werden.

In der Zeichnung zeigen:

Fig. 1      ein Steuergerät gemäß der Erfindung;

Fig. 2      ein Beispiel für den Verlauf des Lenkwinkels x bei Vorliegen einer Unaufmerksamkeit des Fahrers gemäß der Erfindung;

Fig. 3a      den Ablauf des erfindungsgemäßen Verfahrens gemäß einem ersten Ausführungsbeispiel;

Fig. 3b, c      in Verbindung mit Figur 3a den Ablauf des erfindungsgemäßen Verfahrens gemäß einem zweiten Ausführungsbeispiel;

Fig. 4      eine Sigmoid-Funktion;

Fig. 5      ein erstes Ursache-Wirkungs-Diagramm;

Fig. 6      Gauß-Verteilungen für verschiedene Ausprägungsstufen, in welchen ein Wahrscheinlichkeitswert, der die Unaufmerksamkeit des Fahrers repräsentiert, auftreten kann; und

Fig. 7      ein zweites Ursache-Wirkungs-Diagramm.

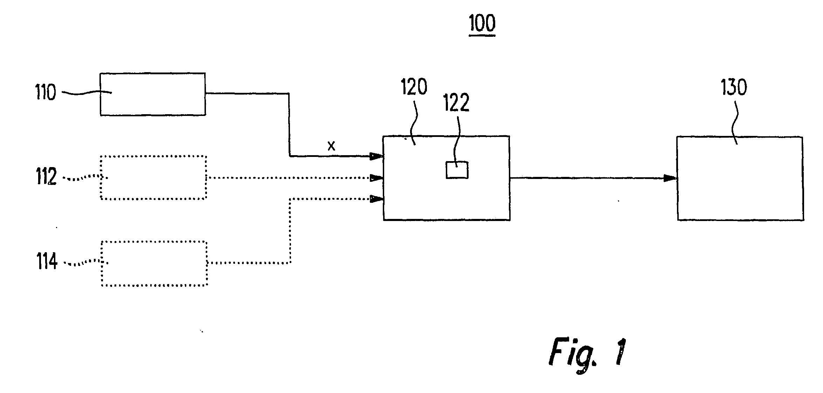

Figur 1 zeigt ein Steuergerät 100 zum Durchführen des erfindungsgemäßen Verfahrens zum Erkennen einer Unaufmerksamkeit des Fahrers eines Fahrzeugs, insbesondere eines Kraftfahrzeugs. Das Steuergerät ist vorzugsweise in dem Fahrzeug (nicht gezeigt) montiert und umfasst einen Lenkradwinkelsensor 110 zum Erfassen des aktuellen Lenkradwinkels x, das heißt der Lenkbewegung, verursacht durch den Fahrer. Darüber hinaus umfasst das Steuergerät 100 eine Steuereinrichtung 120, welche vorzugsweise als Mikrocontroller ausgebildet ist. Die Steuereinrichtung 120 erfasst ein von dem Lenkradwinkelsensor 110 erzeugtes Sensorsignal, welches den Lenkradwinkel x repräsentiert.

Der Lenkradwinkel x repräsentiert einen ersten und erfindungsgemäß bevorzugten Indikator für eine Unaufmerksamkeit des Fahrers. Neben dem Lenkradwinkel kann die Steuereinrichtung 120 grundsätzlich auch noch weitere Sensorsignale von anderen Sensoren 112, 114 als weitere Indikatoren für die Unaufmerksamkeit des Fahrers empfangen und auswerten. Solche weiteren Sensorsignale sollen zunächst unberücksichtigt bleiben, sie finden aber weiter unten in der Beschreibung Erwähnung.

Zum Erkennen einer Unaufmerksamkeit des Fahrers läuft auf der Steuereinrichtung 120 ein Computerprogramm 122 ab, welches die Unaufmerksamkeit gemäß einem erfindungsgemäßen und nachfolgend beschriebenen Verfahren durch Auswerten des Lenkradwinkels x als bevorzugtem Indikator erkennt. Wird eine Unaufmerksamkeit des Fahrers festgestellt, so ist es vorteilhaft, wenn die Steuereinrichtung 120 eine Warneinrichtung 130 ansteuert, damit diese einen akustischen oder optischen Warnhinweis an den Fahrer ausgibt. Aufgrund des Warnhinweises wird dem Fahrer sein unaufmerksames Verhalten beim Führen des Fahrzeugs vergegenwärtigt und ihm Gelegenheit gegeben, seine Aufmerksamkeit wiederherzustellen.

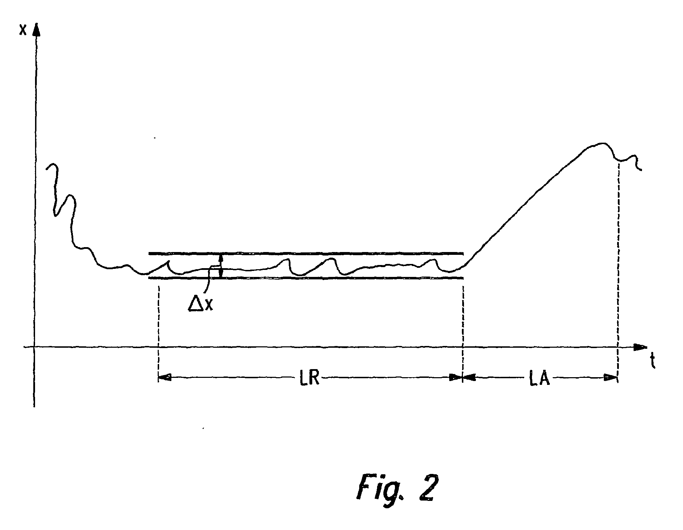

In Figur 2 ist ein typischer Verlauf des Lenkradwinkels aufgezeigt, wie er vorliegt, wenn eine Unaufmerksamkeit des Fahrers mit Hilfe der vorliegenden Erfindung erkannt wird. Dieser Verlauf ist insofern typisch für das Vorliegen einer Unaufmerksamkeit des Fahrers, als dass er zunächst eine Lenkruhephase LR aufweist, in welcher er sich nicht wesentlich ändert; in Figur 2 verbleibt der Lenkwinkel x während der Lenkruhephase LR in dem durch die beiden parallelen horizontalen Linien begrenzten Auslenkungsbereich $\Delta x$. Charakteristisch für das Vorliegen einer Unaufmerksamkeit im Sinne der Erfindung ist dann eine sehr starke beziehungsweise heftige Lenkaktion, die sich an diese Lenkruhephase anschließt. Diese heftige Lenkaktion LA ist in Figur 2 durch den steilen Anstieg des Lenkwinkels x am Ende der Ruhephase repräsentiert.

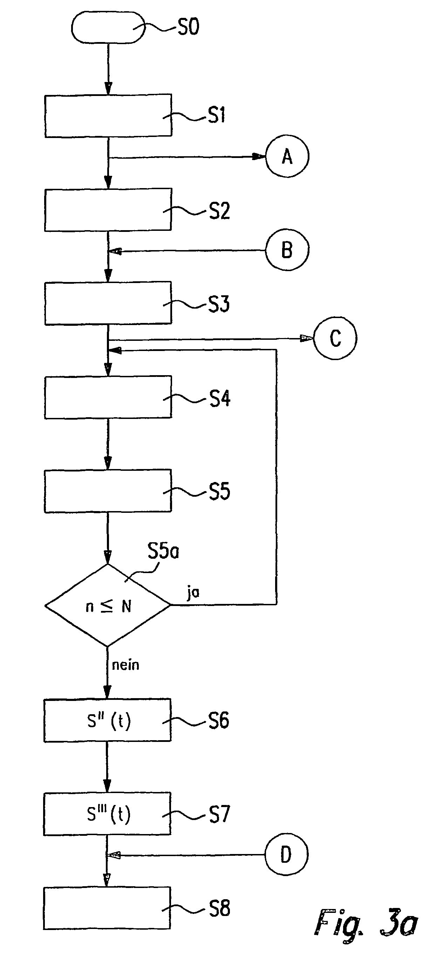

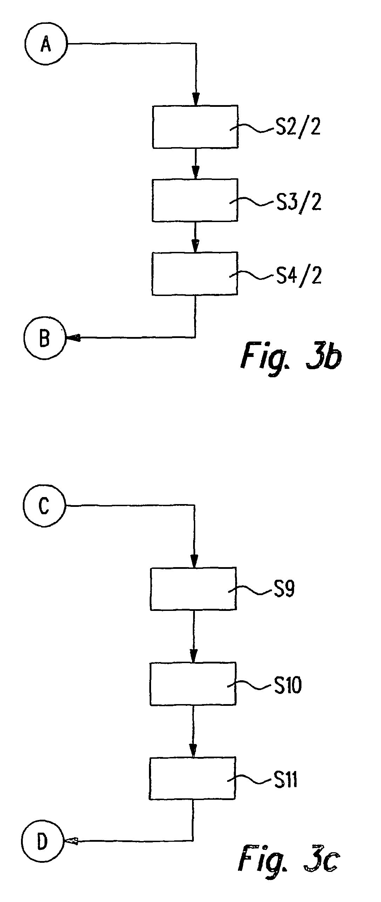

Figur 3 veranschaulicht das erfindungsgemäße, mit Hilfe des oben beschrieben Steuergerätes 100 realisierte, Verfahren. Die in Figur 3a aufgezeigten Verfahrensschritte S0 - S8 repräsentieren zunächst ein erstes Ausführungsbeispiel für dieses Verfahren. Dieses wird nachfolgend zunächst durchgehend und zusammenhängend beschrieben. Für dieses erste Ausführungsbeispiel sind die in Figur 3a gezeigten Abzweigungen A, B, C und D unbeachtlich; sie werden erst relevant bei der Beschreibung eines zweiten Ausführungsbeispiels für das erfindungsgemäße Verfahren, welche sich an die Beschreibung des ersten Ausführungsbeispiels anschließt.

Erstes Ausführungsbeispiel

Nach einem Startschritt S0 sieht das erste Ausführungsbeispiel gemäß Figur 3a zunächst die Erfassung einer Lenkbewegung des Lenkrades des Fahrzeugs, das heißt die Erfassung des Lenkverhaltens des Fahrers in Form des Lenkradwinkels x vor (Verfahrensschritt S1). Auf Basis des erfassten Lenkradwinkels x erfolgt dann in einem zweiten Schritt S2 das Erkennen der Lenkruhephase LR, siehe Figur 2. Ebenfalls in Schritt 2 erfolgt dann die Berechnung eines Varianzverhältnisses $vv(x, t_1)$ als Quotient einer zweiten zu einer ersten Lenkradwinkelvarianz. Dabei berechnet sich die erste Lenkradwinkelvarianz $v(x, t_1 - \Delta t)$ zu einem frühen Zeitpunkt $t_1 - \Delta t$ gemäß folgender Formel (1):

$$v(x, t_1 - \Delta t) = \text{var}(x(t_1 - \Delta t), \ldots, x(t_1 - \Delta t - T)) = \frac{1}{T} \sum_{t=(t_1 - \Delta t)}^{(t_1 - \Delta t - T)} (x(t) - \bar{x})^2 \qquad (1)$$

wobei

$x(t_1 - \Delta t)$    den Lenkradwinkel x zum Zeitpunkt $t_1 - \Delta t$;
$\Delta t$    ein Vielfaches des Abtastintervalls;
$T$    ein Beobachtungszeitfenster;
$\underline{t_1 - \Delta t}$    den Beobachtungszeitpunkt;
$\bar{x}$    einen zeitlichen Mittelwert des Lenkradwinkels x gemittelt über dem Beobachtungszeitfenster T; und
var    die mathematische Varianzfunktion

repräsentiert.

Die zweite Lenkradwinkelvarianz $v(x, t_1)$ berechnet sich gemäß folgender Formel (2):

$$v(x, t_1) = \text{var}(x(t_1), \ldots, x(t_1 - T)) = \frac{1}{T} \sum_{t=(t_1)}^{(t_1 - T)} (x(t) - \bar{x})^2 \qquad (2),$$

wobei die Variablen die gleiche Bedeutung wie bei Formel (1) haben mit dem einzigen Unterschied, dass sie zum Beobachtungszeitpunkt $t_1$ betrachtet werden.

Aus der ersten und zweiten Lenkradwinkelvarianz berechnet sich dann das Varianz-Verhältnis $vv(x, t_1)$ wie folgt:

$$vv(x, t_1) = \frac{v(x, t_1)}{v(x, t_1 - \Delta t)} \qquad (3)$$

Das im Verfahrensschritt S2 berechnete Varianz-Verhältnis repräsentiert deswegen ein zuverlässiges Maß für die Schwere der Unaufmerksamkeit des Fahrers beim Lenken des Fahrzeugs zum Zeitpunkt $t_1$, weil es das typische Lenkverhalten eines Fahrers bei Unaufmerksamkeit wirkungsvoll erfasst. Typisches Lenkverhalten bei Unaufmerksamkeit zeichnet sich - wie bereits oben unter Bezugnahme auf Figur 2 gesagt - durch eine erste Lenkruhephase LR ohne oder mit nur geringer Lenkaktivität und durch eine anschließende zweite Lenkaktionsphase LA mit überdurchschnittlich heftigen Lenkbewegungen aus. Die erste Phase führt zu dem frühen Beobachtungszeitpunkt $t_1 - \Delta t$ zu einem geringen Betrag der ersten Varianz. Dagegen führt die zweite Phase, insbesondere zum Zeitpunkt $t_1$, zu einem erheblich größeren Wert für die zweite Varianz. Insgesamt resultiert daraus ein kleiner Wert für den Nenner und ein großer Wert für den Zähler, so dass aufgrund dieser beiden Effekte insgesamt ein großer Wert für das Varianz-Verhältnis gebildet wird. Solange das Varianz-Verhältnis $\leq 1$ ist, deutet dies darauf hin, dass der Fahrer nicht unaufmerksam ist. Erst wenn das Varianz-Verhältnis einen Wert größer 1 annimmt, deutet dies darauf hin, dass der Fahrer des Fahrzeugs dem Straßenverkehr nicht genügend Aufmerksamkeit widmet.

Der Wert des Varianz-Verhältnisses kann theoretisch beliebig groß werden. Beliebig große Werte dieser Art sind jedoch ungeeignet, um die Unaufmerksamkeit des Fahrers hinreichend genau klassifizieren zu können.

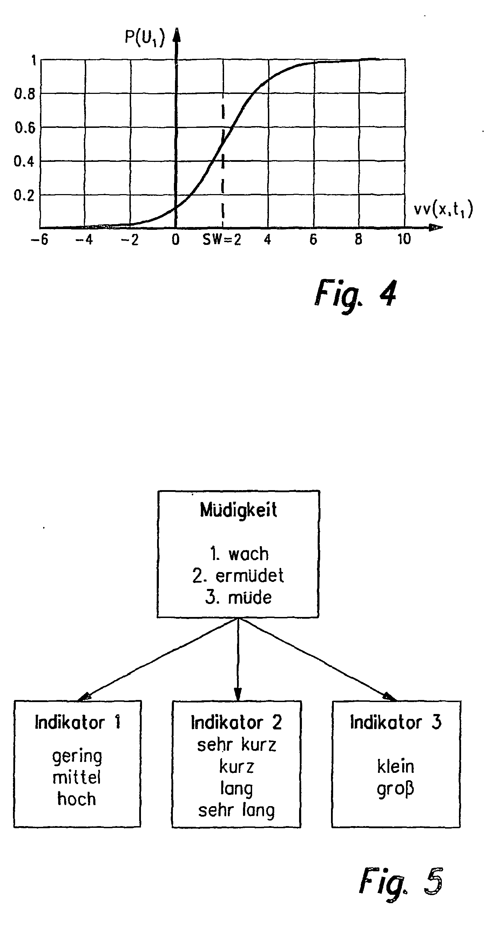

In einer besonderen Variante des erfindungsgemäßen Verfahrens wird deshalb in einem nachfolgenden Verfahrensschritt S3 das Varianz-Verhältnis in einen Wahrscheinlichkeitswert umgerechnet. Diese Umrechnung erfolgt vorzugsweise mit Hilfe einer in Figur 4 dargestellten Sigmoid-Funktion mit einem geeignet vorgegebenen Schwellenwert SW. Bei der in Figur 3 dargestellten Sigmoid-Funktion beträgt dieser Schwellenwert SW = 2. Dies bedeutet, dass bei einem auf der Abszisse in Figur 3 aufgetragenen Varianz-Verhältnis $vv(x, t_1)$ von 2 eine Wahrscheinlichkeit $P(U_n)$ mit n = 1 (erster Indikator) von 0,5, das heißt von 50 %, dafür besteht, dass der Fahrer des Fahrzeugs zum Zeitpunkt $t_1$ unaufmerksam war. Wie aus dem Graphen der Sigmoid-Funktion in Figur 3 zu erkennen ist, kann die Wahrscheinlichkeit $P(U_1)$ grundsätzlich je nach Größe des Varianz-Verhältnisses einen Wert zwischen 0 und 100 % annehmen.

Die Sigmoid-Funktion gewährleistet einen "weichen" Übergang von totaler Aufmerksamkeit entsprechend einem Wahrscheinlichkeitswert für die momentane Unaufmerksamkeit von 0 zu totaler Unaufmerksamkeit, entsprechend einem Wert für die Unaufmerksamkeit von 1 = 100 %. Mathematisch berechnet sich die in Figur 3 dargestellte Sigmoid-Funktion gemäß folgender Formel (4):

$$P(U_1) = \frac{1}{1 + e^{-(vv(x,t_1)-S)}} \qquad (4),$$

wobei

$P(U_1)$     die Wahrscheinlichkeit für die Unaufmerksamkeit des Fahrers beim Lenken des Fahrzeugs repräsentiert, wobei
$U_1$     ein mit Hilfe des ersten Indikators (Lenkradwinkel: n = 1) detektiertes Unaufmerksamkeitsereignis und
$S$     den Schwellenwert

repräsentiert.

Die bisher, das heißt bis einschließlich Verfahrensschritt S3, vorgenommenen Auswertungen des Lenkradwinkels x haben eine Erkenntnis über das Vorhandensein oder Nichtvorhandensein einer Unaufmerksamkeit des Fahrers zum Zeitpunkt $t_1$ ermöglicht. Eine weitere Auswertung der bisher gewonnenen Erkenntnisse, vorzugsweise mit Hilfe eines dynamischen probabilitischen Modells, ermöglicht darüber hinaus Rückschlüsse auf die möglichen Ursachen einer festgestellten Unaufmerksamkeit. Mit den nachfolgend beschriebenen Verfahrensschritten S4 - S6 wird deshalb ein Verfahren aufgezeigt, mit dessen Hilfe beispielsweise festgestellt werden kann, mit welcher Wahrscheinlichkeit Müdigkeit die Ursache für die bei dem Fahrer detektierte Unaufmerksamkeit sein kann.

Diesen soeben beschriebenen Zusammenhang zwischen der Müdigkeit als Ursache für eine daraus resultierende Unaufmerksamkeit beim Lenken des Fahrzeugs ist in Figur 5 veranschaulicht. Die dort eingezeichneten Pfeile zeigen von der Ursache Müdigkeit hin auf verschiedene mögliche Wirkungen, insbesondere eine Unaufmerksamkeit beim Lenken des Fahrzeugs (Indikator 1). Neben der Wirkung Unaufmerksamkeit kann die Müdigkeit jedoch auch noch andere beobachtbare Wirkungen wie zum Beispiel ein gehäuft auftretendes Lidschlussverhalten (Indikator 2) oder eine verzögerte Reaktionsfähigkeit (Indikator 3) haben.

Um aus einem detektierten Unaufmerksamkeitsereignis beim Lenken des Fahrzeugs auf eine Müdigkeit des Fahrers rückschließen zu können, wird erfindungsgemäß in Verfahrensschritt S4 ein erster Wahrscheinlichkeitsvektor $O_{n=1}$ ermittelt, dessen Elemente $O_{n=1,\,kn=1}$ jeweils Wahrscheinlichkeitswerte dafür repräsentieren, dass der Wahrscheinlichkeitswert $P(U_1)$ in einzelnen vorgegebenen und geeignet ausgewählten Ausprägungsstufen $k_n$ mit $k_n \in \{1...K\}$ auftritt. Der Parameter n repräsentiert dabei einen jeweils ausgewerteten Indikator, wobei n = 1 das Lenkverhalten beziehungsweise den Lenkradwinkel als Indikator repräsentiert.

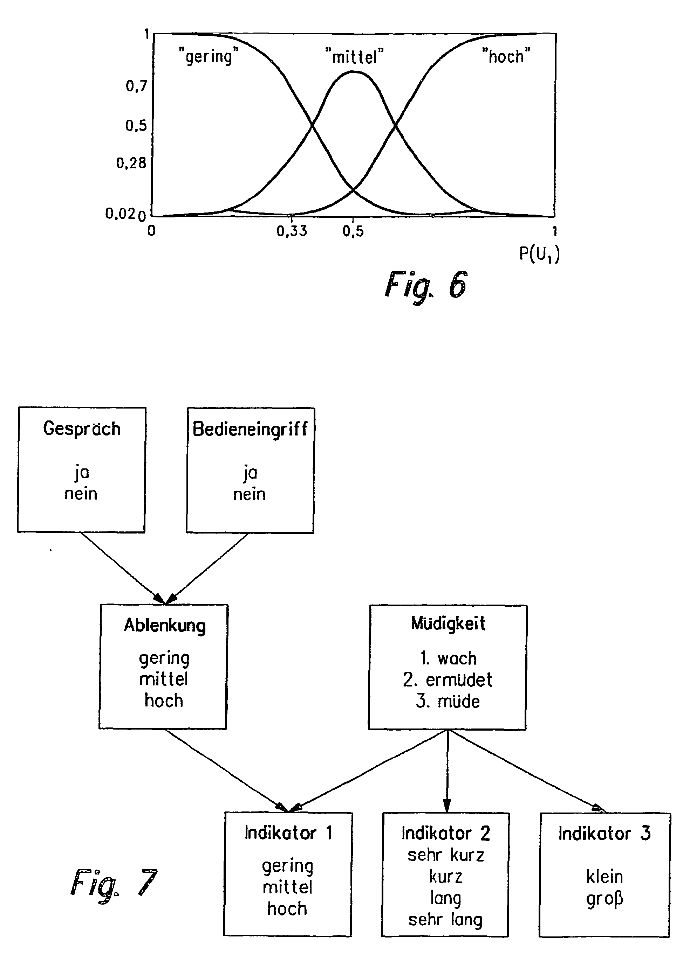

Die Ermittlung des ersten Wahrscheinlichkeitsvektors $O_{n=1}$ ist in Figur 6 veranschaulicht. Dort ist auf der Abszisse der in dem vorherigen Verfahrensschritt S3 ermittelte Wahrscheinlichkeitswert $P(U_1)$ zwischen 0 und 100 % aufgetragen, welcher die Wahrscheinlichkeit für die Unaufmerksamkeit des Fahrers beim Lenken des Fahrzeugs zum

Zeitpunkt $t_1$ repräsentiert. Im Verfahrensschritt S4 soll nun der zuvor ermittelte Wahrscheinlichkeitswert $P(U_1)$ vorgegebenen sein und geeignet definierten Ausprägungsstufen $k_1 \in \{1...K_1\}$ zugeordnet werden. Bei dem in Figur 6 gezeigten Ausführungsbeispiel wurden drei Ausprägungsstufen "gering" ($k_1 = 1$), "mittel" ($k_1 = 2$) und "hoch" ($k_1 = 3$) (vergleiche Figur 5) für den Indikator 1 vorgegeben, die in Figur 6 jeweils durch eine eigene Gauß-Funktion repräsentiert sind. Die Anzahl der Ausprägungsstufen für den Indikator 1 ist in diesem Beispiel somit $K_1 = 3$.

Diese Gauß-Funktionen für die jeweiligen Ausprägungsstufen sind hinsichtlich ihrer Anzahl und Parameter, wie zum Beispiel ihrer Mittelpunkte oder ihrer Varianzen, je nach Anwendungsfall geeignet zu parametrieren. Wie aus Figur 6 ersichtlich, würde nach Durchführung des Verfahrensschrittes S4 einem beispielhaft angenommenen Wahrscheinlichkeitswert $P(U_1)$ von 0,33 für die Unaufmerksamkeit des Fahrers eine Wahrscheinlichkeit von 70 % dafür zugeordnet, dass dieser Wahrscheinlichkeitswert mit nur geringer Ausprägung auftritt. Mit 28 %-iger Wahrscheinlichkeit tritt der berechnete Wahrscheinlichkeitswert mit einer mittleren Ausprägung auf und mit lediglich 2 % tritt der berechnete Wahrscheinlichkeitswert mit einer hohen Ausprägung auf.

Anders ausgedrückt erhält man bei dem in Figur 6 gezeigten Beispiel für den angenommenen Wahrscheinlichkeitswert von 0,33 für die Unaufmerksamkeit des Fahrers zum Zeitpunkt $t_1$ die Aussage, dass die Wahrscheinlichkeit für eine Unaufmerksamkeit des Fahrers mit einer Wahrscheinlichkeit von 70 % nur gering ausgeprägt war, das heißt dass der Fahrer mit einer Wahrscheinlichkeit von 70 % aufmerksam war. Gleichermaßen erhält man die Aussage, dass der Fahrer mit einer Wahrscheinlichkeit von 28 % mittelmäßig aufmerksam war und dass er mit einer Wahrscheinlichkeit von lediglich 2 % hochgradig unaufmerksam war.

In einem nachfolgenden Verfahrensschritt S5 können nun eine beliebige Anzahl von Müdigkeitsstufen definiert werden und bedingte Wahrscheinlichkeiten zwischen diesen Stufen sowie dem beobachteten Lenkunaufmerksamkeitsereignis zugeordnet werden. Bei dem in Figur 5 gezeigten Beispiel sind insgesamt drei Müdigkeitsstufen "wach", "ermüdet" und "müde" vorgesehen. Es können dann, ebenfalls noch in Verfahrensschritt S5, bedingte Wahrscheinlichkeiten in Form einer Matrix B zwischen jeder dieser Müdigkeitsstufen und einer der oben beschriebenen Ausprägungsstufen zugeordnet werden. Diese Matrix B umfasst eine Gesamtzahl von Matrixelementen, welche sich aus dem Produkt Anzahl der Ausprägungsstufen multipliziert mit der Anzahl der vorgegebenen Müdigkeitsstufen berechnet. Bei drei Ausprägungsstufen "gering", "mittel" und "hoch" für das Lenkunaufmerksamkeitsereignis und bei den oben genannten drei Müdigkeitsstufen ergibt sich für die Matrix B eine Gesamtanzahl von 3 * 3 = 9 bedingten Wahrscheinlichkeiten. Eine dieser Wahrscheinlichkeiten gibt beispielsweise an, wie groß die Wahrscheinlichkeit dafür ist, dass ein Lenkunaufmerksamkeitsereignis in der "hohen" Ausprägungsstufe auftritt, gegeben, dass das probabilistische Modell in der ersten Müdigkeitsstufe "wach" ist. Diese bedingten Wahrscheinlichkeiten sind geeignet zu parametrieren.

Mit dem in Schritt S5 ermittelten Wahrscheinlichkeitsvektor $O_{n=1}$ und der berechneten Matrix B ist es nun möglich, in einem Verfahrensschritt S6 einen Müdigkeitswahrscheinlichkeitsvektor S' zu ermitteln, dessen Elemente jeweils Wahrscheinlichkeiten P (Müdigkeitsstufe) dafür repräsentieren, dass die erfasste Unaufmerksamkeit des Fahrers beim Lenken des Fahrzeugs einzelnen vorgegebenen und geeignet ausgewählten Müdigkeitsstufen zugeordnet ist. Die Berechnung des Müdigkeitswahrscheinlichkeitsvektors S' erfolgt gemäß folgender Formel (5):

$$S'(t) = O_1^T \cdot B_1 \qquad\qquad (5),$$

wobei

$O_{n=1}^T$ die Transponierte des ersten Wahrscheinlichkeitsvektors; und

$B_1$ die Matrix B für den erfassten Indikator für Lenkunaufmerksamkeiten, repräsentiert durch den Index 1 repräsentiert.

Die soeben beschriebene Rechenvorschrift (5) zur Berechnung des Müdigkeitswahrscheinlichkeitsvektors S' hat den Nachteil, dass sie lediglich auf einer Auswertung des Lenkradwinkels x als Indikator (n=1) basiert. Andere mögliche beobachtbare Auswirkungen von Müdigkeit, wie sie in Figur 5 durch die Indikatoren 2 (zum Beispiel Lidschlusshäufigkeit) oder Indikator 3 (zum Beispiel Reaktionszeit) angedeutet sind, werden in Formel 5 zur Berechnung des Müdigkeitswahrscheinlichkeitsvektors nicht berücksichtigt.

Es ist jedoch auch möglich, diese Indikatoren 2 und 3 sowie weitere geeignete Indikatoren n, wie zum Beispiel den Gierwinkel des Fahrzeugs, das Abstandsverhalten zum Vorderfahrzeug oder ein Abkommen von einer Fahrspur, sofern messbar, für die Berechnung eines präzisierten Müdigkeitswahrscheinlichkeitsvektors S" heranzuziehen. Die soeben beschriebenen Verfahrensschritte S4 und S5 sind dann jeweils nicht nur für den beobachteten Lenkwinkel als Indikator n = 1, sondern auch für die weiteren gewünschten Indikatoren wie Lidschlussverhalten (n = 2) und/oder Reaktionszeit (n = 3) etc. separat durchzuführen (Verfahrensschritt S5a). Im Rahmen von Verfahrensschritt S4 sind dann jeweils individuell für jeden Indikator n eine individuelle Anzahl von Ausprägungsstufen $k_n$ mit $k_n = 1...K$ festzulegen.

Diese Anzahl der Ausprägungsstufen $k_n$ entspricht dann jeweils der Anzahl der Elemente eines dem jeweiligen Indikator zugeordneten Wahrscheinlichkeitsvektors $O_n$. Diese Elemente $O_{n,kn}$ repräsentieren jeweils Wahrscheinlichkeiten $P(O_{n\_,kn})$ dafür, dass Wahrscheinlichkeitswerte $P(U_n)$ für die anderen Unaufmerksamkeitsindikatoren $n = 2...N$ neben der Lenkunaufmerksamkeit $n = 1$ in den einzelnen für die Indikatoren individuell vorgegebenen und geeignet ausgewählten Ausprägungsstufen $k_n$ auftreten.

Auch für diese weiteren Indikatoren n ist dann entsprechend Verfahrensschritt S5 wieder eine entsprechende Matrix $B_n$ individuell vorzugeben. Aufgrund der dann verfügbaren Daten berechnet sich der präzisierte Müdigkeitswahrscheinlichkeitsvektor S'' in Schritt S6 dann gemäß folgender Formel (6):

$$S''(t) = \prod_{n=1}^{N} O_n^T \cdot B_n \qquad (6),$$

wobei:

n  den n-ten Indikator für die Unaufmerksamkeit des Fahrers

$k_n$  das k'te Element des Vektors $O_n$ beziehungsweise die k'te Ausprägungsstufe für den Indikator n;

$O_n^T$  die Transponierte eines Wahrscheinlichkeitsvektors;

$B_n$  die Matrix von bedingten Wahrscheinlichkeiten zwischen einzelnen vorgegebenen Müdigkeitsstufen und einem durch den Indikator n indizierten Unaufmerksamkeitsereignis; und

N  die Anzahl der verwendeten Indikatorenrepräsentiert.

Bei den bisher beschriebenen beiden Varianten zur Berechnung des Müdigkeitswahrscheinlichkeitsvektors S wurde lediglich von der Schwere eines detektierten Unaufmerksamkeitsereignisses, sei es aufgrund eines detektierten Lenkunaufmerksamkeitsereignisses oder auch aufgrund von zusätzlich detektieren Ereignissen, wie erhöhtem Lidschlussverhalten oder verkürzter Reaktionszeit, auf die am wahrscheinlichsten vorliegende Müdigkeitsstufe rückgeschlossen. Eine weitere Präzisierung dieses Rückschlusses von der beobachteten Unaufmerksamkeit zurück auf eine vorliegende Müdigkeitsstufe als Ursache für diese Unaufmerksamkeit lässt sich dadurch erzielen, dass neben der Schwere des Unaufmerksamkeitsereignisses zusätzlich auch die in einem Zeitschritt zuvor ermittelte wahrscheinlichste Müdigkeitsstufe berücksichtigt wird. Auf diese Weise wird gewährleistet, dass nicht bereits ein einmalig beziehungsweise erstmalig detektiertes Unaufmerksamkeitsereignis sofort zu einem Rückschluss auf eine hohe Müdigkeitsstufe führt. Dies würde der realen Charakteristik von Müdigkeit insofern widersprechen, als dass Müdigkeit grundsätzlich ein Phänomen ist, welches nicht plötzlich auftritt, sondern sich erst im Laufe einer gewissen Zeitdauer langsam aufbaut.

Bei den oben erwähnten und in Figur 5 beschriebenen drei Müdigkeitsstufen sind deshalb die Wechsel von einer Müdigkeitsstufe zu einer anderen mit unterschiedlich bedingten Wahrscheinlichkeiten versehen. Diese bedingten Wahrscheinlichkeiten werden im Rahmen des probabilistischen Modells vorzugsweise in Form einer Matrix A geeignet vorgegeben. Die bedingten Wahrscheinlichkeiten in der Matrix A sollten insbesondere zum Ausdruck bringen, dass ein direkter Übergang zum Beispiel von Müdigkeitsstufe 1 "wach" nach Müdigkeitsstufe 3, "müde" wesentlich unwahrscheinlicher ist als ein direkter Übergang von Stufe 1 nach Stufe 2, "ermüdet", bei der gegenüber Stufe 1 bereits erste Einbußen hinsichtlich der Aufmerksamkeit des Fahrers zu erkennen sind. Die Parametrierung der Matrix A sollte außerdem berücksichtigten, dass die Übergänge von einem wachen in einen Zustand mit größerer Müdigkeit mit anderen bedingten Wahrscheinlichkeiten erfolgen, wie die Übergänge von einem müden in einen wacheren Zustand.

Unter zusätzlicher Berücksichtigung der im Zeitschritt zuvor ermittelten wahrscheinlichsten Müdigkeitsstufe berechnet sich der dadurch nochmals präzisierte Müdigkeitswahrscheinlichkeitsvektor S''' dann im Schritt S7 gemäß der folgenden rekursiven Formel (7):

$$S'''(t_1) = S''(t_1) \cdot A \cdot S'''(t_1 - 1) \qquad (7),$$

wobei

$S''(t_1)$  den präzisierten Müdigkeitsvektor S'' ohne Berücksichtigung der im Zeitschritt zuvor ermittelten wahrscheinlichsten Müdigkeitsstufe;

A  die Matrix der bedingten Wahrscheinlichkeiten zwischen einer Müdigkeitsstufe im letzten Zeitschritt und einer aktuellen Müdigkeitsstufe; und

$S'''(t_1 - 1)$      den präzisierten Müdigkeitsvektor $S'''$ im Zeitschritt $t_1 - 1$

repräsentiert.

Zum Starten der rekursiven Berechnung des präzisierten Müdig- keitswahrscheinlichkeitsvektors $S'''$ gemäß Formel (7) als dritte Variante empfiehlt sich ein Anfangswert von $S'''(0) = (1, 0, 0)^T$.

Anders ausgedrückt bewirkt die präzisere Berechnung des Müdigkeitswahrscheinlichkeitsvektors $S'''$ gemäß Formel (7) eine Glättung des Müdigkeitsvektors über der Zeit beziehungsweise er verhindert, dass ein erstmalig erkanntes Unaufmerksamkeitsereignis sofort zu einer sprunghaften Änderung der als wahrscheinlichste Ursache für das detektierte Unaufmerksamkeitsereignis vermuteten Müdigkeitsstufe führt. Durch geeignete Parametrierung von insbesondere der Matrix A wird weiterhin sichergestellt, dass umgekehrt eine plötzlich festgestellte große Aufmerksamkeit des Fahrers nicht sofort zu einer niedrigen Müdigkeitsstufe führt, wenn unmittelbar zuvor noch eine sehr hohe Müdigkeitsstufe als wahrscheinliche Ursache für eine detektierte Unaufmerksamkeit festgestellt wurde.

Das bisher beschriebene Verfahren zielte stets darauf ab, aufgrund einer erfassten Lenkunaufmerksamkeit sowie optional auch aufgrund weiterer Indikatoren für eine Unaufmerksamkeit des Fahrers auf eine gewisse Müdigkeitsstufe als Ursache für die erfasste Unaufmerksamkeit rückzuschließen. Dies ist in Figur 7 nochmals grafisch veranschaulicht, wo die Pfeile von einer Ursache zu einer Wirkung zeigen. In Figur 7 ist jedoch aufgrund der Pfeilführung auch zu erkennen, dass die Müdigkeit nach dem zugrunde liegenden Modell zwar alleinige Ursache für die Indikatoren 2, das heißt Lidschlussverhalten, und 3, das heißt verkürzte Reaktionszeit, ist, dass sie aber nicht die einzige Ursache für eine detektierte Lenkunaufmerksamkeit sein muss. Vielmehr kann ergänzend oder alternativ zur Müdigkeit auch eine gegebene temporäre Ablenkung des Fahrers Ursache für eine bei ihm detektierte Lenkunaufmerksamkeit sein. Ursache für eine Ablenkung kann wiederum ein zum Beispiel mit dem Beifahrer geführtes Gespräch oder ein vorgenommener Eingriff in ein Bedienelement, zum Beispiel das Radio oder das Handschuhfach des Fahrzeugs sein. Werden neben den bisher genannten Unaufmerksamkeitsereignissen oder Indikatoren zusätzlich auch mögliche Ursachen für eine Ablenkung mit Hilfe geeigneter Sensoren, zum Beispiel eines Mikrofons 112 oder einer Kamera 114 (siehe Figur 1) erfasst, so kann durch Auswertung dieser erfassten Ereignisse mit Hilfe des probabilistischen Modells eine Aussage darüber getroffen werden, mit welcher Wahrscheinlichkeit eine Ablenkung des Fahrers, zum Beispiel aufgrund eines geführten Gespräches oder eines vorgenommenen Bedieneingriffs, und mit welcher Wahrscheinlichkeit Müdigkeit als Ursache für die beobachtete Unaufmerksamkeit angenommen werden kann.

Die mit einer der Varianten des ersten Ausführungsbeispiels detektierte Unaufmerksamkeit des Fahrers wird schließlich mit einem Fehlerkriterium, insbesondere einem jeweils geeignet vorgegebenen Schwellenwert verglichen, um dann nach Maßgabe durch das Ergebnis dieses Vergleiches ein Warnsignal an den Fahrer auszugeben (Verfahrensschritt S8 gemäß Figur 3a).

Zweites Ausführungsbeispiel

Das zweite Ausführungsbeispiel des erfindungsgemäßen Verfahrens ist zwar grundsätzlich unabhängig von dem ersten Ausführungsbeispiel, es weist jedoch einzelne Verfahrensschritte auf, welche identisch sind mit einzelnen Verfahrensschritten aus dem ersten Ausführungsbeispiel. Ein grundsätzlicher Unterschied zwischen dem ersten und zweiten Ausführungsbeispiel besteht darin, dass bei dem ersten Ausführungsbeispiel das Erkennen einer Lenkaktion LA und die Berechnung eines Verknüpfungsergebnisses zwischen der Ausprägung der Lenkruhephase und der Ausprägung der Lenkaktion in Form der Berechnung des Varianzverhältnisses zusammenfallen. Das zweite Ausführungsbeispiel bietet demgegenüber den Vorteil, dass nicht nur die Lenkruhephase LR, sondern auch die anschließende Lenkaktion LA und die erfindungsgemäße Verknüpfung der Ausprägungen dieser beiden Phasen jeweils als separate Verfahrensschritte getrennt durchgeführt werden können, wie dies nachfolgend unter Bezugnahme auf die Figuren 3a, 3b und 3c näher beschrieben wird.

Nach einem Startschritt S0 sieht das zweite Ausführungsbeispiel gemäß Figur 3a zunächst die Erfassung der Lenkbewegung des Lenkrades des Fahrzeugs, das heißt die Erfassung des Lenkverhaltens des Fahrers in Form des Lenkradwinkels x vor (Verfahrensschritt S1) ; insoweit stimmen das erste und das zweite Ausführungsbeispiel noch überein. Für das zweite Ausführungsbeispiel folgt nun nicht der Verfahrensschritt S2 gemäß Figur 3a, sondern das Verfahren verzweigt über die Marke A in die Figur 3b, wo in Verfahrensschritt S2/2 zunächst die Ausprägung der Lenkruhephase ermittelt wird. Die Ausprägung der Lenkruhephase meint insbesondere deren Zeitdauer. Eine Lenkruhephase ist so lange gegeben, so lange der Lenkwinkel des Fahrzeugs innerhalb des vorbestimmten Lenkradwinkelintervalls $\Delta x$ liegt, vgl. Figur 2. Die Zeitdauer, während der diese Situation andauert, repräsentiert dann die Ausprägung der Lenkruhephase LR.

Nachfolgend wird in einem Verfahrensschritt S3/2 die Ausprägung einer sich an die detektierte Lenkruhephase anschließenden Lenkaktion detektiert. Dazu wird der dann auftretende maximale Gradient des Lenkwinkels ermittelt. In Figur 2 ist dieser Gradient in Form der Steigung des Lenkwinkels veranschaulicht, wie er auftritt, nachdem der Lenkradwinkel das Lenkradwinkelintervall $\Delta x$ verlassen hat.

In Verfahrensschritt S4/2 werden dann die Ausprägung der Lenkruhephase und der Lenkaktion über einen mehrdimensionalen Operator miteinander verknüpft. Bei dem mehrdimensionalen Operator kann es sich um ein Kennfeld, eine Gewichtungsfunktion oder eine logische Entscheidungsfunktion handeln. Das Ergebnis dieser Anwendung des mehrdimensionalen Operators repräsentiert dann ein geeignetes Maß für die Schwere der Unaufmerksamkeit des Fahrers beim Lenken des Fahrzeugs. Vorzugsweise wird die Verknüpfung der beiden genannten Ausprägungen jedoch nur dann durchgeführt, wenn sich in den zuvor durchgeführten Schritten S2/2 oder S3/2 gezeigt hat, dass die Ausprägung der Lenkruhephase in Form ihrer Zeitdauer größer ist als eine vorbestimmte Mindestzeitdauer und der maximale Gradient des Lenkradwinkels einen vorbestimmten Gradientenschwellenwert überschreitet. Andernfalls werden die Ausprägungen der Lenkruhephase und der Lenkaktion von dem erfindungsgemäßen Verfahren nicht als hinreichend stark genug ausgeprägt angesehen, um aus ihrem kombinierten Vorliegen auf eine Unaufmerksamkeit des Fahrers schließen zu können.

Der mehrdimensionale Operator wird vorzugsweise nach Maßgabe durch die aktuelle Geschwindigkeit des Fahrzeugs und/oder nach Maßgabe durch die Dynamik des Fahrstils des Fahrers des Fahrzeugs dimensioniert. Konkret bedeutet dies, dass bei der Auswertung der detektierten Ausprägungen der Lenkruhephase und der Lenkaktion berücksichtigt wird, dass die Lenkbewegungen bei hohen Geschwindigkeiten des Fahrzeugs typischerweise geringer sind als bei niedrigen Geschwindigkeiten. Die Anpassung an den Fahrstil des Fahrers verhindert, dass eine zum Beispiel von einem Rallyefahrer absichtlich durchgeführte hektische Lenkbewegung in Verbindung mit einer zuvor festgestellten vermeintlichen Lenkruhephase fälschlicherweise als eine Unaufmerksamkeit des Fahrers interpretiert wird.

Das in Verfahrensschritt S4/2 ermittelte Verknüpfungsergebnis gemäß dem zweiten Ausführungsbeispiel kann nun auf verschiedene Weise weiter ausgewertet und verarbeitet werden.

Eine erste Möglichkeit besteht darin, dass dieses mit Hilfe zum Beispiel der Sigmoid-Funktion normalisiert wird. Diese Möglichkeit ist in Figur 3b durch die Marke B angezeigt, die auf den Eingang von Verfahrensschritt S3 in Figur 3a verzweigt. Es kann dann nicht nur der Verfahrensschritt S3, sondern es können dann auch alle weiteren Verfahrensschritte S4 bis S8 wie oben unter Bezugnahme auf Figur 3a beschrieben, durchgeführt werden.

Eine zweite Möglichkeit zur Weiterverarbeitung des in Verfahrensschritt S4/2 gewonnenen Verknüpfungsergebnisses besteht darin, dass es zwar noch mit Hilfe der Sigmoid-Funktion in Verfahrensschritt S3 normiert wird, dann aber nicht gemäß Verfahrensschritt S4 ff. weiter ausgewertet, sondern wie in Figur 3a durch die Marke C angedeutet, in Verfahrensschritt S9 gemäß Figur 3c weiterverarbeitet wird. Mit diesem Schritt wird angedeutet, dass die zuvor durchgeführte Berechnung des Verknüpfungsergebnisses, sei es gemäß dem ersten Ausführungsbeispiel in Schritt 2 oder gemäß dem zweiten Ausführungsbeispiel in Schritt S4/2, mehrfach während eines vorbestimmten Messzeitintervalls durchgeführt werden sollte. Die wiederholte Durchführung der Berechnung der Verknüpfungsergebnisse zu verschiedenen Zeitpunkt ti mit i = 1-I während des Messzeitintervalls führt dazu, dass am Ende des Messzeitintervalls vorzugsweise eine Vielzahl von Verknüpfungsergebnissen vorliegen. Diese Verknüpfungsergebnisse werden vorzugsweise, wie bisher beschrieben, am Ausgang von Verfahrensschritt S3 abgegriffen, weil sie dann in normierter Form vorliegen. Alternativ können aber auch die unmittelbar in den Verfahrensschritten S2 und S4/2 gewonnenen Verknüpfungsergebnisse direkt in Verfahrensschritt S9 gesammelt und gespeichert werden. In Verfahrensschritt S10 werden diese Verknüpfungsergebnisse dann individuell gewichtet, indem jedem dieser Ergebnisse ein Gewichtungsfaktor zugeordnet wird. Diese Gewichtungsfaktoren repräsentieren die jeweilige Fahrsituation des Fahrzeugs oder die aktuelle Ablenkung des Fahrers jeweils zu dem Zeitpunkt, auf den sich das Verknüpfungsergebnis bezieht.

In Verfahrensschritt S11 wird dann schließlich ein gewichtetes Verknüpfungsergebnis durch mathematische, vorzugsweise arithmetische gewichtete Mittelwertbildung der während des Messzeitintervalls gewonnenen Verknüpfungsergebnisse unter Berücksichtigung von deren zugeordneten Gewichtungsfaktoren berechnet.

Die Gewichtungsfaktoren werden unter Berücksichtigung der Tageszeit, das heißt circadianer Einflussfaktoren und/oder der Zeit seit Fahrtbeginn festgelegt. Mit dem gewichteten Ergebnis der Verknüpfung liegt ein sehr zuverlässiges und vor allen Dingen relativ einfach und schnell zu ermittelndes Maß für die Schwere der Unaufmerksamkeit des Fahrers beim Fahren des Fahrzeugs vor. Dieses gemittelte Ergebnis der Verknüpfung wird dann vorzugsweise wie in Figur 3c in Verbindung mit Figur 3a über die Marke D in Verfahrensschritt S8 einem Fehlerkriterium unterzogen und zur Generierung eines Warnsignals an den Fahrer ausgewertet. Das Fehlerkriterium ist dann erfüllt, wenn die Summe aller in den letzten x Minuten berechneten Verknüpfungsergebnisse jeweils gewichtet mit ihren individuellen Gewichtungsfaktoren einen vorgegebenen Schwellenwert überschreitet. Die bisher beschriebene zweite Möglichkeit zur Weiterverarbeitung des in Verfahrensschritt S4/2 gemäß dem zweiten Ausführungsbeispiel gewonnenen Verknüpfungsergebnisses durch die Schritte S9 bis S11 besteht gleichermaßen auch für das in Verfahrensschritt S2 gemäß dem ersten Ausführungsbeispiel gewonnenen Verknüpfungsergebnis.

Eine dritte Möglichkeit zur Weiterverarbeitung des Verknüpfungsergebnisses aus Schritt S2 oder aus Schritt S4/2 besteht in einer vorzugsweise zeitlich parallelen Ausführung der Schritte S4 bis S7 und S9 bis S11.

Die beiden Ausführungsbeispiele des erfindungsgemäßen Verfahrens in allen ihren Varianten werden vorzugsweise in Form mindestens eines Computerprogramms realisiert. Das Computerprogramm kann gegebenenfalls zusammen mit weiteren Computerprogrammen auf einem Datenträger abgespeichert sein. Bei dem Datenträger kann es sich um eine Diskette, eine Compact Disc, einen sogenannten Flash-Memory oder dergleichen handeln. Das auf dem Datenträger abgespeicherte Computerprogramm kann dann als Produkt an einen Kunden verkauft werden. Alternativ zu einer Übertragung per Datenträger steht auch die Möglichkeit einer Übertragung über ein Kommunikationsnetzwerk, insbesondere das Internet.

**Patentansprüche**

**1.** Verfahren zum Erkennen, wann der Fahrer eines Fahrzeugs, insbesondere eines Kraftfahrzeugs, unaufmerksam wird, umfassend folgende Schritte:

- Erfassen einer Bewegung eines Lenkrades des Fahrzeugs in Form eines Lenkradwinkels x (Verfahrensschritt S1); und
- Erkennen einer Lenkruhephase und Ermitteln der Größe der Ausprägung der Lenkruhephase durch Auswerten des erfassten Lenkradwinkels und/oder dessen zeitlicher Änderung;
- Erkennen einer sich an die Lenkruhephase anschließenden Lenkaktion und Ermitteln der Größe der Ausprägung der Lenkaktion durch Auswerten der zeitlichen Änderung des Lenkradwinkels; und
- Ermitteln eines Maßes für die Schwere der Unaufmerksamkeit des Fahrers beim Lenken des Fahrzeugs durch Bewerten des Ergebnisses einer Verknüpfung der Ausprägung der Lenkruhephase und der Ausprägung der Lenkaktion

**gekennzeichnet durch**

- Durchführen der Verknüpfung zu verschiedenen Zeitpunkten ti während eines vorbestimmten Messzeitintervalls;
- Speichern der Ergebnisse der Verknüpfungen zu den Zeitpunkten ti jeweils zusammen mit zugeordneten Gewichtungsfaktoren, welche die Fahrsituation des Fahrzeugs oder die aktuelle Ablenkung des Fahrers jeweils zum Zeitpunkt ti repräsentieren; und
- Berechnen eines gewichteten Ergebnisses der Verknüpfung durch mathematische, vorzugsweise arithmetische Mittelwertbildung der während des Messzeitintervalls gespeicherten Ergebnisse unter Berücksichtigung von deren zugeordneten Gewichtungsfaktoren.

**2.** Verfahren nach Anspruch 1,
**dadurch gekennzeichnet,**
**dass** die Gewichtungsfaktoren unter Berücksichtigung circadianer Einflussfaktoren und/oder der Zeit seit Fahrtbeginn berechnet werden

**3.** verfahren nach Anspruch 1 oder 2,
**gekennzeichnet durch**
Ausgeben einer Information, insbesondere einer akustischen oder optischen Warnmeldung an den Fahrer des Fahrzeugs, wenn das vorzugsweise gewichtete Ergebnis einen vorbestimmten Schwellenwert überschreitet.

**4.** Verfahren nach Anspruch 1, 2 oder 3,
**dadurch gekennzeichnet,**
**dass**
die Ausprägung der Lenkruhephase ermittelt wird zum Zeitpunkt $t_1$-$\Delta t$ in Form einer ersten Lenkradwinkelschwankung und/oder zum Zeitpunkt $t_1$ in Form einer zweiten Lenkradwinkelschwankung, jeweils nach Maßgabe des erfassten Lenkradwinkels x.

**5.** Verfahren nach Anspruch 4,
**dadurch gekennzeichnet,**
**dass**
- sich die erste Lenkradwinkelschwankung in Form einer Lenkradwinkelvarianz $v(x, t_1$-$\Delta t)$ gemäß folgender Formel (1) berechnet:

$$v(x, t_1 - \Delta t) = \mathrm{var}(x(t_1 - \Delta t), \ldots, x(t_1 - \Delta t - T)) = \frac{1}{T} \sum_{t=(t_1 - \Delta t)}^{((t_1 - \Delta t) - T)} (x(t) - \bar{x})^2 \qquad (1)$$

wobei

x($t_1$-$\Delta$t) den Lenkradwinkel x zum Zeitpunkt $t_1$-$\Delta$t;
$\Delta$t ein Vielfaches des Abtastintervalls;
T ein Beobachtungszeitfenster;
$t_1$-$\Delta$t den Beobachtungszeitpunkt;
x einen zeitlichen Mittelwert des Lenkradwinkels x gemittelt über dem Beobachtungszeitfenster T; und
var die mathematische Varianzfunktion

repräsentiert; und

- sich die zweite Lenkradwinkelschwankung in Form einer Lenkradwinkelvaxianz v(x,$t_1$) gemäß folgender Formel (2) berechnet:

$$v(x, t_1) = \mathrm{var}(x(t_1), \ldots, x(t_1 - T)) = \frac{1}{T} \sum_{t=(t_1)}^{(t_1 \cdot T)} (x(t) - \bar{x})^2 \qquad (2),$$

wobei die Parameter dieselbe Bedeutung haben wie bei der Formel (1).

6. Verfahren nach Anspruch 5,
   **dadurch gekennzeichnet,**
   **dass**
   die Ausprägung der Lenkaktion sowie die Verknüpfung von Lenkruhephase und Lenkaktion ermittelt werden durch Bilden eines Schwankungs-Verhältnisses vv(x, $t_1$), vorzugsweise als Quotient von der zweiten zu der ersten Lenkradwinkelvarianz.

7. Verfahren nach Anspruch 6,
   **dadurch gekennzeichnet,**
   **dass** sich das Varianz-Verhältnis vv(x, $t_1$) gemäß folgender Formel (3) berechnet:

$$vv(x, t_1) = \frac{v(x, t_1)}{v(x, t_1 - \Delta t)} \qquad . \qquad (3)$$

8. Verfahren nach Anspruch 1,
   **dadurch gekennzeichnet,**
   **dass** die Ausprägung der Lenkruhephase ermittelt wird als diejenige Zeitdauer, während derer der Lenkradwinkel innerhalb eines vorbestimmten Lenkradwinkelintervalls ($\Delta$x) bleibt.

9. Verfahren nach Anspruch 8,
   **dadurch gekennzeichnet,**
   **dass** das Lenkradwinkelintervall nach Maßgabe durch die aktuelle Geschwindigkeit des Fahrzeugs vorbestimmt wird.

10. Verfahren nach Anspruch 4, 5, 8 oder 9,
    **dadurch gekennzeichnet,**
    **dass** die Ausprägung der Lenkaktion im Anschluss an eine vorangegangene Lenkruhephase in Form des dann auftretenden maximalen Gradienten des Lenkradwinkels ermittelt wird.

**11.** Verfahren nach Anspruch 10,
**dadurch gekennzeichnet,**
**dass** die Verknüpfung der Ausprägung der Lenkruhephase und der Ausprägung der Lenkaktion zu einem Zeitpunkt t1 durch einen mehrdimensionalen Operator erfolgt, vorzugsweise jedoch nur dann, wenn die Ausprägung der Lenkruhephase in Form ihrer Zeitdauer größer ist als eine vorbestimmte Mindestzeitdauer und der maximale Gradient des Lenkradwinkels einen vorbestimmten Gradientenschwellenwert überschreitet.

**12.** Verfahren nach Anspruch 11,
**dadurch gekennzeichnet,**
**dass** der mehrdimensionale Operator ein Kennfeld, eine Gewichtungsfunktion oder eine logische Entscheidungsfunktion repräsentiert.

**13.** Verfahren nach Anspruch 11 oder 12,
**dadurch gekennzeichnet,**
**dass** der mehrdimensionale Operator nach Maßgabe durch die Geschwindigkeit des Fahrzeugs und/oder eine Dynamik des Fahrstils des Fahrers des Fahrzeugs dimensioniert wird.

**14.** verfahren nach einem der Ansprüche 6,7 oder 11,12,13,
**dadurch gekennzeichnet,**
**dass** in einem nachfolgenden Schritt (Verfahrensschritt S3) das Ergebnis der Verknüpfung in Form des Varianz-Verhältnis vv(x, t) oder des mehrdimensionalen Operators vorzugsweise mit Hilfe der Sigmoid-Funktion auf einen Wahrscheinlichkeitswert $P(U_1)$ zwischen 0 und 100 % abgebildet wird, welcher die Unaufmerksamkeit des Fahrers beim Lenken des Fahrzeugs zum Zeitpunkt $t_1$ repräsentiert.

**15.** Verfahren nach Anspruch 14
**gekennzeichnet durch**
folgende weitere Schritte zum Beurteilen der Müdigkeit des Fahrers:

- Ermitteln eines ersten Wahrscheinlichkeitsvektors $O_{n-1}$, dessen Elemente $O_{n-1, k1}$ jeweils Wahrscheinlichkeitswerte $P(O_{1,k1})$ dafür repräsentieren, dass ein Wahrscheinlichkeitswert $P(U_1)$ in einzelnen vorgegebenen und ausgewählten Ausprägungsstufen $k_1$ mit $k_1 \in \{1...K_1\}$ auftritt (Verfahrensschritt S4); und
- Ermitteln eines Müdigkeitswahrscheinlichkeitsvektors S', dessen Elemente jeweils Wahrscheinlichkeiten P (Müdigkeitsstufe) dafür repräsentieren, dass die erfasste Unaufmerksamkeit des Fahrers beim Lenken des Fahrzeugs einzelnen vorgegebenen und geeignet ausgewählten Müdigkeitsstufen zugeordnet ist, gemäß fol-gender Formel (5):

$$S'(t) = O_I^T \cdot B_1; \qquad\qquad (5),$$

wobei

$O_I^T$ die Transponierte des ersten Wahrscheinlichkeitsvektors;

$B_1$ die Matrix B vorgegebene bedingte Wahrscheinlichkeiten bezüglich der Lenkunaufmerksamkeit, repräsentiert **durch** den Indikator n = 1; und
$K_1$ die Anzahl der Ausprägungsstufen für den Indikator n = 1

repräsentiert.

**16.** Verfahren nach Anspruch 13,
**gekennzeichnet durch**
folgende weitere Schritte:

- Ermitteln von weiteren wahrscheinlichkeitsvektoren $O_{n-2} ... O_{n=N}$, deren Elemente $O_{n,kn}$ mit $k_n = 1 ... K_n$ jeweils

Wahrscheinlichkeiten $P(O_{n,kn})$ dafür repräsentieren, dass Wahrscheinlichkeitswerte $P(U_n)$ für andere Unaufmerksamkeitsindikatoren $n = 2...N$ des Fahrers, neben der Lenkunaufmerksamkeit $n = 1$, insbesondere das Lidschlussverhalten $n = 2$ oder die Reaktionszeit $n = 3$, in einzelnen, für die Unaufmerksamkeitsindikatoren individuell vorgegebenen Ausprägungsstufen $k_n$ auftreten, und

- sich der Müdigkeitswahrscheinlichkeitsvektor S'' in Verfahrensschritt S6 dann gemäß folgender Formel (6) berechnet:

$$S''(t) = \prod_{n=1}^{N} O_n^T \cdot B_n \qquad (6),$$

wobei

n den n'ten Indikator für die Unaufmerksamkeit des Fahrers;

$O_n^T$ die Transponierte der weiteren Wahrscheinlichkeitsvektoren;

$B_n$ die Matrix B für den Indikator n; und
N die Anzahl der Indikatoren

repräsentiert.

**17.** Verfahren nach einem der Ansprüche 15 oder 16, **gekennzeichnet durch**

- Speichern des Müdigkeitswahrscheinlichkeitsvektors S'''(t-1); und
- Berechnen eines weiterhin präzisierten Müdigkeitswahrscheinlichkeitsvektors S'''(t) gemäß folgender Formel (7)(Verfahrensschritt S7):

$$S'''(t) = S''(t) \cdot A \cdot S'''(t-1), \qquad (7)$$

wobei

A die Matrix der bedingten Wahrscheinlichkeiten zwischen einer Müdigkeitsstufe aus dem letzten Zeitschritt und einer aktuellen Müdigkeitsstufe

repräsentiert.

**18.** Verfahren nach einem der Ansprüche 16 oder 17,
**dadurch gekennzeichnet,**
**dass** neben der Lenkunaufmerksamkeit und den optionalen weiteren Indikatoren für die Unaufmerksamkeit des Fahrers auch festgestellt wird, ob der Fahrer ein Gespräch führt oder einen Eingriff in ein Bedienelement, zum Beispiel das Radio oder das Handschuhfach des Fahrzeugs tätigt; und dass durch Auswertung dieser erfassten Ereignisse mit Hilfe des probabilistischen Modells eine Aussage darüber getroffen werden kann, mit welcher Wahrscheinlichkeit eine Ablenkung des Fahrers aufgrund des Gesprächs oder des Bedieneingriffs und mit welcher Wahrscheinlichkeit Müdigkeit des Fahrers als Ursache für die beobachtete Unaufmerksamkeit angenommen werden kann.

**19.** Computerprogramm (122) mit Programmcode für ein Steuergerät zum Erkennen einer Unaufmerksamkeit eines Fahrers eines Fahrzeugs,
**dadurch gekennzeichnet,**
**dass** der Programmcode ausgebildet ist zur Durchführung des Verfahrens nach einem der Ansprüche 1 - 19.

**20.** Datenträger
**gekennzeichnet durch**

das Computerprogramm nach Anspruch 19.

**Claims**

1. Method for identification when the driver of a vehicle, in particular of a motor vehicle, is not paying attention, comprising the following steps:

   - detection of any movement of a steering wheel of the vehicle in the form of a steering wheel angle x (method step S1); and
   - identification of a steering quiescent phase and determination of the magnitude of the extent of the steering quiescent phase by evaluation of the detected steering wheel angle and/or its rate of change;
   - identification of a steering action following the steering quiescent phase and determination of the magnitude of the extent of the steering action by evaluation of the rate of change of the steering wheel angle; and
   - determination of a measure of the severity of the inattentiveness by the driver while steering the vehicle by assessment of the result of a link between the extent of the steering quiescent phase and the extent of the steering action

   **characterized by**

   - carrying out the logical operation at different times ti during a predetermined measurement time interval;
   - storage of the results of the logical operations relating to the times ti in each case together with the associated weighting factors which represent the driving situation of the vehicle or the current distraction of the driver, in each case relating to the time ti; and
   - calculation of a weighted result of the logical operation by mathematical, preferably arithmetic, averaging of the results stored during the measurement time interval, taking into account the weighting factors associated with them.

2. Method according to Claim 1,
   **characterized in that**
   the weighting factors are calculated taking into account circadian influencing factors and/or the time since the journey started.

3. Method according to Claim 1 or 2,
   **characterized by**
   the outputting of information, in particular an audible or visual warning message to the driver of the vehicle, when the preferably weighted result exceeds a predetermined threshold value.

4. Method according to Claim 1, 2 or 3,
   **characterized in that**
   the extent of the steering quiescent phase is determined for the time $t_1-\Delta t$ in the form of a first steering wheel angle fluctuation and/or for the time $t_1$ in the form of a second steering wheel fluctuation, in each case based on the detected steering wheel angle x.

5. Method according to Claim 4,
   **characterized in that**

   - the first steering wheel angle fluctuation is calculated in the form of a steering wheel angle variance $v(x, t_1-\Delta t)$ using the following formula (1):

$$v(x, t_1-\Delta t) = \mathrm{var}(x(t_1-\Delta t), \ldots, x(t_1-\Delta t-T)) = \frac{1}{T} \sum_{t=(t_1-\Delta t)}^{((t_1-\Delta t)-T)} (x(t) - \bar{x})^2$$

(1)

where:

x($t_1$-$\Delta$t) represents the steering wheel angle x at the time $t_1$-$\Delta$t;
$\Delta$t represents a multiple of the sampling interval;
T represents an observation time window;
$t_1$-$\Delta$t represents the observation time;
x represents a time mean value of the steering wheel angle x averaged over the observation time window T; and
var represents the mathematical variance function;

and
the second steering wheel angle fluctuation in the form of a steering wheel angle variance v(x, $t_1$) is calculated using the following formula (2):

$$v(x,t_1) = \text{var}(x(t_1),\ \ldots,\ x(t_1-T)) = \frac{1}{T}\sum_{t=(t_1)}^{(t_1-T)}(x(t) - \bar{x})^2$$

(2)

where the variables have the same meanings as in the formula (1).

**6.** Method according to Claim 5,
**characterized**
**in that**
the extent of the steering action as well as the linking of the steering quiescent phase and the steering action are determined by formation of a fluctuation ratio vv(x, $t_1$), preferably as the quotient of the second steering wheel angle variance divided by the first.

**7.** Method according to Claim 6,
**characterized**
**in that** the variance ratio vv(x, $t_1$) is then calculated in accordance with the following formula (3):

$$vv(x,t_1) = \frac{v(x,t_1)}{v(x,t_1 - \Delta t)}\ .$$

(3)

**8.** Method according to Claim 1,
**characterized**
**in that** the extent of the steering quiescent phase is determined as that time period during which the steering wheel angle remains within a predetermined steering wheel angle interval ($\Delta$x).

**9.** Method according to Claim 8,
**characterized**
**in that**
the steering wheel angle interval is predetermined on the basis of the current speed of the vehicle.

**10.** Method according to Claim 4, 5, 8, or 9,
**characterized**
**in that**
the extent of the steering action following a previous steering quiescent phase is determined in the form of the maximum gradient of the steering wheel angle which then occurs.

**11.** Method according to Claim 10,
**characterized**
**in that**

the link between the extent of the steering quiescent phase and the extent of the steering action at a time t1 is produced by means of a multidimensional operator, but preferably only when the extent of the steering quiescent phase in the form of its time period is greater than a predetermined minimum time period and the maximum gradient of the steering wheel angle exceeds a predetermined gradient threshold value.

**12.** Method according to Claim 11,
   **characterized**
   **in that**
   the multidimensional operator represents a family of characteristics, a weighting function or a logical decision function.

**13.** Method according to Claim 11 or 12,
   **characterized**
   **in that**
   the multidimensional operator is dimensioned on the basis of the speed of the vehicle and/or dynamics of the driving style of the driver of the vehicle.

**14.** Method according to one of Claims 6, 7 or 11, 12, 13,
   **characterized**
   **in that**,
   in a subsequent step (method step S3), the result of the logical operation is mapped in the form of the variance ratio $vv(x, t)$ or of the multidimensional operator, preferably with the aid of the sigmoid function, onto a probability value $P(U_1)$ between 0 and 100%, which represents the inattentiveness by the driver in the steering of the vehicle at the time $t_1$.

**15.** Method according to Claim 14,
   **characterized by**
   the following further steps for assessment of the fatigue of the driver:

   - determination of a first probability vector $O_{n=1}$, whose elements $O_{n=1, k1}$ each represent probability values $P(O_{1, k1})$, of a probability value $P(U_1)$ occurring in individual, predetermined and selected extent levels $k_1$ where $k_1 \, c \, \{1...K_1\}$ (method step S4); and
   - determination of a fatigue probability vector S', whose elements each represent probabilities P (fatigue level), of the detected inattentiveness by the driver in steering of the vehicle being associated with individual, predetermined and suitably selected fatigue levels, using the following formula (5):

$$S'(t) \; = \; O_1^T \; \cdot \; B_1; \hspace{4cm} (5),$$

   with

   $O^T_1$ representing the transpose of the first probability vector;
   $B_1$ the matrix B representing predetermined conditional probabilities with respect to the steering inattentiveness, represented by the indicator n = 1; and
   $K_1$ representing the number of extent levels for the indicator n = 1.

**16.** Method according to Claim 13,
   **characterized by**
   the following further steps:

   - determination of further probability vectors $O_{n=2}...O_{n=N}$ whose elements $O_{n, kn}$ were $k_n=1...K_n$ each represent probabilities $P(O_{n, kn})$ of the probability values $P(U_n)$ occurring for other inattentiveness indicators n = 2...N for the driver, in addition to the steering inattentiveness n = 1, in particular the eyelid closure behavior n = 2 or the reaction time n = 3, in individual extent levels $k_n$, which are predetermined individually for the inattentiveness indicators, and
   - the fatigue probability vector S'' in the method step S6 then being calculated using the following formula (6):

$$S''(t) = \prod_{n=1}^{N} O_n^T \cdot B_n \qquad\qquad (6),$$

where

N represents the n-th indicator for the inattentiveness by the driver;

$O_n^T$  represents the transpose of the further probability vectors;

$B_n$ represents the matrix B for the indicator n; and
N represents the number of indicators.

**17.** Method according to one of Claims 15 or 16,
**characterized by**

- storage of the fatigue probability vector S'''(t-1) ; and
- calculation of a more precise fatigue probability vector S''' (t) using the following formula (7) (method step S7):

$$S'''(t) = S''(t) \cdot A \cdot S'''(t-1), \qquad\qquad (7)$$

where

A represents the matrix of the conditional probabilities between a fatigue level from the last time step and a current fatigue level.

**18.** Method according to one of Claims 16 or 17,
**characterized**
**in that**, in addition to the steering inattentiveness and the optional further indicators for the inattentiveness by the driver, the method also determines whether the driver is holding a conversation or is using a control element, for example is operating the radio or the glove compartment in the vehicle; and wherein these detected events can be evaluated with the aid of the probabilistic model in order to make a statement about the probability with which it can be assumed that the driver has been distracted, on the basis of the conversation or the control action, and the probability of driver fatigue being the cause of the observed inattentiveness.

**19.** Computer program (122) with program code for a controller for identification of inattentiveness by a driver of a vehicle,
**characterized**
**in that**
the program code is designed to carry out method according to one of Claims 1-18.

**20.** Data storage medium
**characterized by**
the computer program according to Claim 19.

**Revendications**

**1.** Procédé pour détecter si le conducteur d'un véhicule, en particulier d'un véhicule automobile, est inattentif, lequel procédé comprend les étapes qui consistent à :

- détecter un déplacement du volant du véhicule sous la forme d'un angle de volant x (étape de procédé S1),
- détecter une phase de repos du volant et déterminer le niveau d'importance de la phase de repos du volant par évaluation de l'angle de volant détecté et/ou de ses modifications dans le temps,
- détecter une action sur le volant qui suit la phase de repos du volant et déterminer le niveau d'importance de l'action sur le volant par évaluation de la modification de l'angle de volant dans le temps et
- déterminer le niveau de gravité de l'inattention du conducteur dans sa conduite du véhicule par évaluation du résultat d'une association entre l'importance de la phase de repos du volant et l'importance de l'action sur le volant,

**caractérisé en ce que**

- l'association est réalisée à différents instants $t_i$ pendant une durée de mesure prédéterminée,
- les résultats des associations aux instants $t_i$ sont conservés en mémoire en même temps que des facteurs associés respectifs de pondération qui représentent la situation de conduite du véhicule ou la conduite effective par le conducteur à chaque instant $t_i$ et
- un résultat pondéré de l'association est calculé par la formation d'une valeur moyenne mathématique et de préférence arithmétique des résultats conservés en mémoire pendant la période de mesure en tenant compte des facteurs de pondération qui leur sont associés.

2.  Procédé selon la revendication 1, **caractérisé en ce que** les facteurs de pondération sont calculés en tenant compte de facteurs circadiens et/ou de la durée qui s'est écoulée depuis le début de la conduite.

3.  Procédé selon les revendications 1 ou 2, **caractérisé en ce qu'**une information, en particulier un avertissement acoustique ou visuel, est délivrée au conducteur du véhicule lorsque le résultat de préférence pondéré dépasse une valeur de seuil prédéterminée.

4.  Procédé selon les revendications 1, 2 ou 3, **caractérisé en ce que** l'importance de la phase de repos du volant est déterminée l'instant $t_1 - \Delta t$ sous la forme d'une première variation de l'angle de volant et/ou à l'instant $t_1$ sous la forme d'une deuxième variation de l'angle de volant, chaque fois en fonction de l'angle de volant x détecté.

5.  Procédé la revendication 1, **caractérisé en ce que**

- la première variation de l'angle de volant est calculée sous la forme d'une variance $v(x, t_1 - \Delta t)$ de l'angle de volant par la formule (1) ci-dessous :

$$v(x, t_1 - \Delta t) = \mathrm{var}(x(t_1 - \Delta t), ..., x(t_1 - \Delta t - T)) = \frac{1}{T} \sum_{t=(t_1 - \Delta t)}^{((t_1 - \Delta t) - T)} (x(t) - \bar{x})^2 \qquad (1)$$

dans laquelle

$x(t_1 - \Delta t)$ représente l'angle de volant x à l'instant $t_1 - \Delta t$,
$\Delta t$ représente un multiple de l'intervalle d'échantillonnage,
T représente une fenêtre temporelle d'observation,
$t_1 - \Delta t$ représente l'instant d'observation,
$\bar{x}$ représente la valeur moyenne de l'angle de volant x sur la fenêtre temporelle d'observation T et
var représente la fonction mathématique de variance

et

- la deuxième variation de l'angle de volant est calculée sous la forme de la variance $v(x, t_1)$ de l'angle de volant selon la formule (2) :

$$v(x, t_1) = \mathrm{var}(x(t_1), ..., x(t_1 - T)) = \frac{1}{T} \sum_{t=(t_1)}^{(t_1 - T)} (x(t) - \bar{x})^2 \qquad (2),$$

dans laquelle les paramètres ont la même signification que dans la formule (1).

**6.** Procédé selon la revendication 5, **caractérisé en ce que** l'importance de l'action sur le volant ainsi que l'association entre la phase de repos du volant et l'action sur le volant sont déterminées en formant un rapport de variation $vv(x, t_1)$, de préférence sous la forme du quotient entre la deuxième et la première variance d'angle de volant.

**7.** Procédé selon la revendication 6, **caractérisé en ce que** le rapport de variance $vv(x, t_1)$ est calculé selon la formule (3) ci-dessous :

$$vv(x,t_1) = \frac{v(x,t_1)}{v(x,t_1 - \Delta t)} \quad . \qquad\qquad (3)$$

**8.** Procédé selon la revendication 1, **caractérisé en ce que** l'importance de la phase de repos du volant est déterminée comme étant la durée pendant laquelle l'angle de volant reste à l'intérieur d'un intervalle prédéterminé $\Delta x$ d'angle de volant.

**9.** Procédé selon la revendication 8, **caractérisé en ce que** l'intervalle d'angle de volant est prédéterminé en fonction de la vitesse effective du véhicule.

**10.** Procédé selon les revendications 4, 5, 8 ou 9, **caractérisé en ce que** l'importance de l'action sur le volant après une phase précédente de repos du volant est déterminée sous la forme du gradient maximum observé de l'angle de volant.

**11.** Procédé selon la revendication 10, **caractérisé en ce que** l'association entre l'importance de la phase de repos du volant et l'importance de l'action sur le volant à un instant $t_1$ s'effectue par un opérateur pluridimensionnel, de préférence uniquement lorsque l'importance de la phase de repos du volant exprimée par sa durée est plus grande qu'une durée minimale prédéterminée et que le gradient maximum du volant dépasse une valeur de seuil de gradient prédéterminée.

**12.** Procédé selon la revendication 11, **caractérisé en ce que** l'opérateur pluridimensionnel est un champ de caractéristiques, une fonction de pondération ou une fonction logique de décision.

**13.** Procédé selon les revendications 11 ou 12, **caractérisé en ce que** l'opérateur pluridimensionnel est dimensionné en fonction de la vitesse du véhicule et/ou de la dynamique du style de conduite du conducteur du véhicule.

**14.** Procédé selon l'une des revendications 6, 7 ou 11, 12, 13, **caractérisé en ce que** dans une étape suivante (étape de procédé S3), le résultat de l'association exprimé sous la forme du rapport du rapport de variance $vv(x, t)$ ou de l'opérateur pluridimensionnel est formé de préférence en appliquant la fonction sigmoïde sur une valeur de probabilité $P(U_1)$ comprise entre 0 et 100 % qui représente l'inattention du conducteur lors de sa conduite du véhicule à l'instant $t_1$.

**15.** Procédé selon la revendication 14, **caractérisé par** les autres étapes ci-dessous destinées à évaluer la fatigue du conducteur :

- détermination d'un premier vecteur de probabilité $O_{n=1}$, dont les éléments $O_{n-1,k1}$ représentent chacun des valeurs de probabilité $P(O_{1, k1})$ qui indiquent qu'une valeur de probabilité $P(U_1)$ survient dans des niveaux d'importance prédéterminés et sélectifs $k_1$, avec $k_1 \in \{1 ... K_1\}$ (étape de procédé S4) et
- détermination d'un vecteur S' de probabilité de fatigue dont les éléments représentent chacun des probabilités $P$ (niveau de fatigue) qui indiquent que l'inattention détectée du conducteur dans sa conduite du véhicule est associée à différents niveaux de fatigue prédéterminés et sélectionnés de manière appropriée, selon la formule (5) ci-dessous :

$$S'(t) = O_1^T \cdot B_1; \qquad\qquad (5),$$

dans laquelle :

$U_1$ représente la transposée du premier vecteur de probabilité,

$B_1$ représente la matrice B prédéterminée des probabilités d'inattention de conduite, représentée par l'indicateur n = 1 et

$K_1$ représente le nombre des niveaux d'importance de l'indicateur n = 1.

**16.** Procédé selon la revendication 13, **caractérisé par** les autres étapes ci-dessous :

- détermination d'autres vecteurs de probabilité On=2 ... $O_{n=N}$, dont les éléments $O_{n,kn}$ avec $k_n$=1 ... $K_n$ représentent chacun des probabilités $P(O_{n,kn})$ que des valeurs de probabilité $P(U_n)$ d'autres indicateurs n = 2 ... N d'inattention du conducteur surviennent en plus de l'inattention de conduite n = 1, en particulier la fermeture des paupières n = 2 ou la durée de réaction n = 3, dans différentes niveaux d'importance $k_n$ prédéterminés individuellement pour les indicateurs d'inattention et
- le vecteur S" de probabilité de fatigue est calculé dans l'étape de procédé S6 par la formule (6) ci-dessous :

$$S''(t) = \prod_{n=1}^{N} O_n^T \cdot B_n$$

dans laquelle

n représente le nième indicateur d'inattention du conducteur,

$O_i^T$ représente la transposée des autres vecteurs de probabilité,

$B_n$ représente la matrice B de l'indicateur n et
N représente le nombre des indicateurs.

**17.** Procédé selon l'une des revendications 15 ou 16, **caractérisé par** les étapes qui consistent à :

- conserver en mémoire le vecteur de probabilité de fatigue S'''(t-1) et
- calculer un vecteur encore plus précis S'''(t) de probabilité de fatigue par la formule (7) ci-dessous (étape de procédé S7) :

$$S'''(t) = S''(t) \cdot A \cdot S'''(t-1), \qquad (7)$$

dans laquelle

A représente la matrice des probabilités entre un niveau de fatigue de la dernière étape temporelle et le niveau de fatigue actuel.

**18.** Procédé selon l'une des revendications 16 ou 17, **caractérisé en ce qu'**en plus de l'inattention de conduite et des autres indicateurs facultatifs d'inattention du conducteur, on détermine également si le conducteur tient une conversation ou agit sur un élément de commande, par exemple la radio ou le vide-poche du véhicule, et **en ce que** par évaluation des événements ainsi détectés, on peut tirer à l'aide du modèle probabilistique une conclusion sur la probabilité que l'inattention du conducteur soit provoquée par une conversation ou une action et la probabilité que la fatigue du conducteur est la cause de l'inattention observée.

**19.** Programme informatique (122) doté d'un code de programme pour un appareil de commande qui détecte l'inattention du conducteur d'un véhicule, **caractérisé en ce que** le code de programme est configuré en vue de l'exécution du procédé selon l'une des revendications 1 à 19.

**20.** Support de données **caractérisé par** le programme informatique selon la revendication 19.

*Fig. 1*

*Fig. 2*

Fig. 3a

S0

S1

A

S2

B

S3

C

S4

S5

S5a

$n \leq N$

ja

nein

$S''(t)$ — S6

$S'''(t)$ — S7

D

S8

A → S2/2 → S3/2 → S4/2 → B

**Fig. 3b**

C → S9 → S10 → S11 → D

**Fig. 3c**

**Fig. 4**

**Fig. 5**

**Fig. 6**

**Fig. 7**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19818239 A1
- US 6061610 A
- DE 2546345
- EP 0147539 A2
- JP 7093678 A